# EUROPÄISCHE PATENTSCHRIFT

(11) **EP 2 280 922 B1**
(45) Veröffentlichungstag und Bekanntmachung des Hinweises auf die Patenterteilung: **06.05.2015**
(21) Anmeldenummer: 09734255.4
(22) Anmeldetag: 11.03.2009
(51) Int. Cl.: C07B 63/00, C07C 51/43, C07C 57/04

(54) **VERFAHREN ZUR HERSTELLUNG UND AUFREINIGUNG WÄSSRIGER PHASEN**
METHOD FOR PRODUCING AND PURIFYING AQUEOUS PHASES
PROCÉDÉ DE FABRICATION ET DE LAVAGE DE PHASES AQUEUSES

(30) Priorität: 24.04.2008 DE 102008020688
(43) Veröffentlichungstag der Anmeldung: 09.02.2011
(73) Patentinhaber: Evonik Degussa GmbH, 45128 Essen (DE)
(72) Erfinder: HENGSTERMANN, Axel, 48308 Senden (DE); KUPPINGER, Franz-Felix, 45768 Marl (DE); LEISTNER, Jörg, 33378 Rheda-Wiedenbrück (DE); MOSLER, Jürgen, 45772 Marl (DE); JANSENS, Peter, 6137 KV Sittard (NL)
(86) Internationale Anmeldenummer: PCT/EP2009/052831
(87) Internationale Veröffentlichungsnummer: WO 2009/130085

(56) Entgegenhaltungen:
- DE-A1- 10 149 353
- US-A- 4 780 568
- US-A1- 2003 175 159
- US-A1- 2004 256 319

## Beschreibung

Die vorliegende Erfindung ist auf ein Verfahren zur Aufreinigung einer ein Zielprodukt und Wasser beinhaltenden, ungereinigten Phase, auf ein Verfahren zur Herstellung einer aufgereinigten Phase beinhaltend ein Zielprodukt und Wasser, auf eine aufgereinigte Phase beinhaltend ein Zielprodukt und Wasser, auf ein Verfahren zur Herstellung eines auf (Meth)Acrylsäure basierenden Polymers, auf Polymere, erhältlich durch dieses Verfahren, auf chemische Produkte wie etwa Fasern oder Formkörper sowie auf die Verwendung von Polymeren gerichtet.

Bei der Herstellung industriell relevanter Zielprodukte, wie beispielsweise (Meth)Acrylsäure, fallen im Verlaufe der Herstellung häufig wässrige Zusammensetzungen an, welche neben dem gewünschten Zielprodukte und Wasser auch zahlreiche Verunreinigungen enthalten. Bevor (Meth)Acrylsäure als Edukt bei der Herstellung von Polymeren durch radikalische Polymerisation eingesetzt werden kann, war es bisher üblich, die (Meth)Acrylsäure aus derartigen wässrigen Zusammensetzungen zu isolieren, wobei im Falle der Acrylsäure häufig eine Reinheit von mehr als 99 Gew.-% angestrebt wird. Bei der Herstellung von Poly-(Meth)acrylaten aus einer so gewonnenen, hochreinen (Meth)Acrylsäure wird diese dann wieder in Wasser als Lösungsmittel gelöst und in Gegenwart radikalischer Initiatoren polymerisiert.

Als eine Möglichkeit zur Herstellung von hochreinen, organischen Substanzen, wie beispielsweise hochreiner Acrylsäure, ist die Kristallisation zu nennen. Technisch kommen dabei insbesondere zwei Verfahren zur Anwendung, die Suspensionskristallisation und die Schichtkristallisation (Wintermantel et al., Chem. Ing. Tech. 1991, 63,881-891; Steiner et al, Chem. Ing. Tech. 1985, 57, 91-102).

Oft reicht allerdings ein Kristallisationsschritt alleine nicht aus, um Nebenprodukte hinreichend gut aus oder von den Kristallen zu entfernen, da Mikroeinschlüsse von Mutterlaugen oder der Einbau von Verunreinigungen an Kristallfehlstellen etc. unter endlichen Kristallwachstumsbedingungen nicht aufzuschließen sind. Insbesondere das Anhaften von Mutterlauge auf dem Kristall kann die Reinheit der Produkte verschlechtern.

Aus diesem Grund werden die erzeugten Kristalle häufig, insbesondere im Falle einer Kristallsuspension, nach der Trennung von der Mutterlauge mit Waschflüssigkeiten gewaschen und/oder die Kristalle werden bei der Schicht- bzw. Suspensionskristallisation einem Schwitz- bzw. Waschprozess unterzogen, bei dem Verunreinigungen jeglicher Art ggf. abgereichert werden können. Kontinuierlich kann ein solcher Prozess in sogenannten Waschkolonnen durchgeführt werden. Eine Übersicht bietet hier die Dissertation von Poschmann (Zur Suspensionskristallisation organischer Schmelzen und Nachbehandlung der Kristalle durch Schwitzen und Waschen, Diss. Uni. Bremen, Shaker Verlag, Aachen 1996).

Ein derartiges Verfahren, bei dem (Meth)Acrylsäure-Kristalle aus einer wässrigen Schmelze beinhaltend (Meth)Acrylsäure, Wasser sowie von (Meth)Acrylsäure und Wasser verschiedene Verunreinigungen auskristallisiert und die so erhaltenen (Meth)Acrylsäure-Kristalle zumindest teilweise mit einer Kristallschmelze gewaschen werden, ist beispielsweise der WO-A-03/078378 zu entnehmen. Gemäß der Lehre dieser Druckschrift wird (Meth)Acrylsäure aus einer solchen wässrigen Lösung bei einer Temperatur im Bereich von -5 bis 30°C kristallisiert, die so erhaltenen Kristalle anschließend bei einer Temperatur im Bereich von 10 bis 15°C aufgeschmolzen und die (Meth)Acrylsäure-Kristalle anschließend mit der Kristallschmelze gewaschen werden.

Der Nachteil des in der WO-A-03/078378 beschriebenen Verfahrens besteht jedoch unter anderem darin, dass das dort erhaltene Kristallbett zu einer starken Verdichtung neigt, so dass ein für den großtechnischen Einsatz insbesondere bei der Verwendung kontinuierlich betriebener Waschkolonnen problematisch hoher hydraulischer Druck eingesetzt werden muss. Auch weist das in der WO-A-03/078378 beschriebene Verfahren den Nachteil auf, dass zum Aufschmelzen der (Meth)acrylsäure vergleichsweise viel Energie aufgewendet werden muss.

Allgemein liegt der vorliegenden Erfindung die Aufgabe zu Grunde, die sich aus dem Stand der Technik ergebenden Nachteile durch die Bereitstellung geeigneter technischer Lehren zu überwinden.

Insbesondere soll ein Verfahren zur Aufreinigung einer ein Zielprodukt, insbesondere (Meth)Acrylsäure, sowie Wasser beinhaltenden, ungereinigten Phase angegeben werden, welches mit Vorrichtungen bzw. Vorrichtungsbestandteilen durchgeführt werden kann, die im Vergleich zu den aus dem Stand der Technik bekannten Vorrichtungen bzw. Vorrichtungsbestandteilen mit möglichst wenigen, technisch aufwendigen Einbauten versehen sind.

Auch lag der vorliegenden Erfindung die Aufgabe zugrunde, ein Verfahren zur Aufreinigung einer ein Zielprodukt, insbesondere (Meth)Acrylsäure sowie Wasser beinhaltenden, ungereinigten Phase anzugeben, welches aus energetischer Sicht möglichst kostengünstig betrieben werden kann.

Auch sollte dieses Verfahren zur Aufreinigung einer ein Zielprodukt, insbesondere (Meth)Acrylsäure sowie Wasser beinhaltenden, ungereinigten Phase ein Kristallisationsverfahren sein, welches derart effizient durchgeführt werden kann, dass eine erneute Kristallisation der bei der ersten Kristallisation erhaltenen Mutterlauge nicht erforderlich ist.

Darüber hinaus lag der vorliegenden Erfindung die Aufgabe zugrunde, ein Verfahren zur Herstellung eines auf (Meth)Acrylsäure basierenden Polymers anzugeben, welches möglichst kostengünstig betrieben werden kann. Insbesondere sollte es dieses Verfahren ermöglichen, auf eine Aufreinigung von (Meth)acrylsäure bis zu einer Reinheit von mehr als 90 Gew.-% verzichten zu können.

Diese Aufgaben werden gelöst durch ein Verfahren zur Aufreinigung einer ungereinigten Phase beinhaltend ein Zielprodukt, wobei das Zielprodukt eine organische Verbindung ist, welche in einem Zwei-Komponenten-System aus Wasser und der organischen Verbindung ein eutektisches Gemisch zu bilden vermag, vorzugsweise (Meth)Acrylsäure, Wasser und mindestens eine vom Zielprodukt und von Wasser verschiedene Verunreinigung zu einer gereinigten Phase, aufweisend eine Verfahrensstufe, welche folgende Verfahrensschritte beinhaltet:
a) das Zielprodukt und Wasser werden aus der ungereinigten Phase unter Bildung einer Suspension, beinhaltend eine Mutterlauge und Kristalle auskristallisiert, wobei dieses Auskristallisieren bei einer Temperatur T₁ im Bereich von Tₑ - 153°C ≤ T₁ ≤ Tₑ + 3°C (Tₑ = eutektische Temperatur der ungereinigten Phase), besonders bevorzugt bei einer Temperatur T₁ im Bereich von Tₑ - 11°C ≤ T₁ ≤ Tₑ + 2°C, noch mehr bevorzugt bei einer Temperatur T₁ im Bereich von Tₑ - 7°C ≤ T₁ ≤ Tₑ + 1°C und am meisten bevorzugt bei einer Temperatur T₁ im Bereich von Tₑ - 3°C ≤ T₁ ≤ Tₑ erfolgt;
b) die Kristalle werden zumindest teilweise von der Mutterlauge abgetrennt;
c) mindestens ein Teil der abgetrennten Kristalle wird zu einer Schmelze aufgeschmolzen, wobei dieses Aufschmelzen bei einer Temperatur T₂ im Bereich von T₁ < T₂ ≤ T₁ + 20°C, besonders bevorzugt bei einer Temperatur im Bereich von T₁ < T₂ ≤ T₁ + 10°C, noch mehr bevorzugt bei einer Temperatur im Bereich von T₁ < T₂ ≤ T₁ + 5°C und am meisten bevorzugt bei einer Temperatur im Bereich von T₁ < T₂ ≤ T₁ + 1°C erfolgt; und
d) mindestens ein Teil der Schmelze wird dem Schritt b) zurückgeführt, wobei der nicht zurückgeführte Teil der Schmelze als eine gereinigte Phase vorliegt.

Ist demnach Tₑ im Falle einer Acrylsäure-beinhaltenden, ungereinigten Phase beispielsweise gleich -11°C, so erfolgt die Kristallisation in einem Bereich von -26°C bis -8°C, besonders bevorzugt in einem Bereich von -22°C bis -9°C, noch mehr bevorzugt in einem Bereich von -18°C bis -10°C, und am meisten bevorzugt in einem Bereich von -14°C bis -11°C. Erfolgt die Kristallisation bei -11°C, so erfolgt das Aufschmelzen bei einer Temperatur oberhalb von - 11°C bis hinauf zu +9°C, besonders bevorzugt bei einer Temperatur oberhalb von - 11°C bis hinauf zu - 1°C, noch mehr bevorzugt bei einer Temperatur oberhalb von - 11°C bis hinauf zu - 6°C und am meisten bevorzugt bei einer Temperatur oberhalb von - 11°C bis hinauf zu -10°C.

Überraschenderweise wurde festgestellt, dass sich Zusammensetzungen im wesentlichen basierend auf Wasser und einem Zielprodukt, welche zusätzlich unerwünschte Verunreinigungen enthalten, in besonders einfacher und vor allem kostengünstiger Weise aufreinigen lassen, indem derartige Zusammensetzungen auf Temperaturen um den eutektischen Punkt hinab abgekühlt und auf diese Weise Wasserkristalle und Kristalle des Zielproduktes gebildet werden. Diese Kristalle werden von der verbleibenden Mutterlauge abgetrennt und zumindest teilweise bei Temperaturen, die nur unwesentlich höher sind als die Kristallisationstemperatur, aufgeschmolzen, wobei die nicht aufgeschmolzenen Kristalle mit einem Teil dieser Schmelze gewaschen werden. Der nicht zum Waschen eingesetzte Teil der Schmelze stellt eine aufgereinigte Phase aus Wasser und dem Zielprodukt dar, welche im Vergleich zur ursprünglich eingesetzten, ungereinigten Phase hinsichtlich der darin enthaltenen Verunreinigungen abgereinigt ist.

Dieses Verfahren ist insbesondere Vorteilhaft bei Zielprodukten, welche im Verlaufe ihrer Herstellung aus entsprechenden Vorläuferverbindungen irgendwann als wässrige Phase beinhaltend das Zielprodukt, Wasser sowie weitere Verunreinigungen vorliegen und welche später bei der Weiterverarbeitung zu Nachfolgeprodukten ebenfalls in Form wässriger Lösungen eingesetzt werden. Mittels des erfindungsgemäßen Verfahrens kann nämlich darauf verzichtet werden, die Zielprodukte zum Zwecke der Abtrennung von Nebenprodukten zunächst in nahezu vollständig aufgereinigter Form (und somit befreit von Wasser) herzustellen und sie dann bei der Weiterverarbeitung zu Nachfolgeprodukten wieder in Wasser zu lösen, wie dieses derzeit beispielsweise im Falle der Herstellung von Acrylsäure und der nachfolgenden Herstellung von Polyacrylaten aus Acrylsäure üblich ist.

"(Meth)Acrylsäure" wird in diesem Text für die Verbindungen mit den Nomenklaturnamen "Methacrylsäure" und "Acrylsäure" verwendet. Von beiden Verbindungen ist die Acrylsäure erfindungsgemäß bevorzugt. Unter dem Begriff "Zielprodukt" wird erfindungsgemäß eine organische Verbindung verstanden, welche in einem Zwei-Komponenten-System aus Wasser und der organischen Verbindung ein eutektisches Gemisch ("Eutektikum") zu bilden vermag.

Obwohl im Falle von (Meth)Acrylsäure als Zielprodukt die ungereinigte Phase im Vergleich zu dem aus der WO-A-03/078378 beschriebenen Verfahren stärker gekühlt wird, ist bei dem nunmehr bereitgestellten, erfindungsgemäßen Verfahren die zum Aufschmelzen der so entstehenden Kristalle aufzuwendende Energiemenge geringer, da die in der Nähe des eutektischen Punkts liegenden Kristalle bereits bei über -11°C schmelzen. Ansonsten müsste bei herkömmlicher, beispielsweise aus der WO-A-03/078378 bekannter Fahrweise auf Temperaturen von mindestens 0°C wieder aufgewärmt werden, was, wie eingangs erörtert, zu Problemen bei einer kontinuierlich betriebenen Waschkolonne führt.

Im Schritt a) der im erfindungsgemäßen Verfahren enthaltenen Verfahrensstufe werden zunächst das Zielprodukt und Wasser aus der ungereinigten Phase unter Bildung einer Suspension, beinhaltend eine Mutterlauge und Kristalle, insbesondere Kristalle des Zielproduktes und Wasser-Kristalle, auskristallisiert.

Als Zielprodukt kommen alle dem Fachmann bekannten Verbindungen in Betracht, welche in einem Zwei-Komponenten-System mit Wasser ein eutektisches Gemisch zu bilden vermögen und welche im Verlaufe ihrer großtechnischen Herstellung aus entsprechenden Ausgangsverbindungen irgendwann in Form einer wässrigen Phase beinhaltend dieses Zielprodukt, Wasser sowie von diesem Zielprodukt und Wasser verschiedene Verunreinigungen vorliegen.

Erfindungsgemäß besonders bevorzugt als Zielprodukt sind ungesättigte Mono- oder Dicarbonsäuren, wobei unter diesen Acrylsäure und Methacrylsäure als Zielprodukt besonders bevorzugt und Acrylsäure am meisten bevorzugt ist.

Gemäß einer ersten besonderen Ausführungsform des erfindungsgemäßen Verfahrens beinhaltet die ungereinigte Phase als eine wasserreiche Zusammensetzung Acrylsäure in einer Konzentration in einem Bereich von 40 bis 75 Gew.-%, besonders bevorzugt 50 bis 70 Gew.-% und am meisten bevorzugt 55 bis 65 Gew.-%, Wasser in einer Konzentration in einem Bereich von 20 bis 55 Gew.-%, besonders bevorzugt 28 bis 48 Gew.-% und am meisten bevorzugt 34 bis 44 Gew.-%, und von Wasser und Acrylsäure verschiedene Verunreinigungen in einer Konzentration von höchstens 15 Gew.-%, besonders bevorzugt höchstens 10 Gew.-%, noch mehr bevorzugt höchstens 5 Gew.-% und am meisten bevorzugt höchstens 1 Gew.-%, jeweils bezogen auf die Zusammensetzung. Eine solche, ungereinigte Phase kann beispielsweise aus einem Quenchabsorber stammen. Weiterhin kann es sich bei der ungereinigten Phase um eine Acrylsäure abgereicherte Mutterlauge oder das Filtrat aus einer Kristallisation handeln, deren Acrylsäure weiterhin aufgereinigt werden soll. Auch kann eine solche ungereinigte Phase durch den gezielten Zusatz einer der in der ungereinigten Phase enthaltenen Komponente, beispielsweise durch den gezielten Zusatz von Wasser, zu einer Phase, in der die einzelnen Komponenten nicht in den vorstehend genannten Konzentrationsbereichen enthalten sind, erhalten werden, so dass nach gezielter Zugabe dieser Komponenten eine ungereinigte Phase erhalten wird, welche Acrylsäure, Wasser sowie von Acrylsäure und Wasser verschiedene Verunreinigungen in den vorstehend genannten Konzentrationsbereichen enthält. Gemäß einer besonderen Variante dieser ersten bevorzugten Ausführungsform des erfindungsgemäßen Verfahrens beinhaltet die im Verfahrensschritt a) eingesetzte, ungereinigte Phase 58 bis 62 Gew.-% Acrylsäure, 37 bis 41 Gew.-% Wasser sowie 1 bis 2 Gew.-%, jeweils bezogen auf die ungereinigte Phase, von Acrylsäure und Wasser verschiedene Verunreinigungen.

Gemäß einer zweiten besonderen Ausführungsform des erfindungsgemäßen Verfahrens beinhaltet die ungereinigte Phase als eine wasserreiche Zusammensetzung Methacrylsäure in einer Konzentration in einem Bereich von 2 bis 40 Gew.-%, besonders bevorzugt 5 bis 25 Gew.-%, noch mehr bevorzugt 8 bis 20 Gew.-% und am meisten bevorzugt 10 bis 15 Gew.-%, Wasser in einer Konzentration in einem Bereich von 60 bis 98 Gew.-%, besonders bevorzugt 75 bis 95 Gew.-%, noch mehr bevorzugt 80 bis 92 Gew.-% und am meisten bevorzugt 85 bis 90 Gew.-%, und von Wasser und Methacrylsäure verschiedene Verunreinigungen in einer Konzentration von höchstens 10 Gew.-%, besonders bevorzugt höchstens 5 Gew.-%, noch mehr bevorzugt höchstens 1 Gew.-% und am meisten bevorzugt höchstens 0,1 Gew.-%, jeweils bezogen auf die Zusammensetzung. Gemäß einer besonderen Variante dieser zweiten bevorzugten Ausführungsform des erfindungsgemäßen Verfahrens beinhaltet die im Verfahrensschritt a) eingesetzte, ungereinigte Phase 9 bis 14 Gew.-% Methacrylsäure, 85 bis 90 Gew.-% Wasser sowie 1 bis 2 Gew.-%, jeweils bezogen auf die ungereinigte Phase, von Methacrylsäure und Wasser verschiedene Verunreinigungen. Eine solche ungereinigte Phase wird beispielsweise als Ablauf im Quenchabsorber bei der Herstellung von Methacrylsäure erhalten.

Bei der Kristallisation sind als Kristallisationsmittel vorzugsweise solche zu verwenden, die es gestatten, den erfindungsgemäßen Aufreinigungsprozeß kontinuierlich zu gestalten. Vorzugsweise kommt die Suspensionskristallisation zum Einsatz. Diese kann vorteilhafterweise in einem Rührkesselkristallisator, Kratzkristallisator, Kühlscheibenkristallisator, Kristallisierschnecke, Trommelkristallisator, Rohrbündelkristallisator o. dgl. durchgeführt werden. Insbesondere können die in WO-A-99/14181 genannten Kristallisationsvarianten für den genannten Zweck herangezogen werden. Von besonderem Vorteil sind hier wiederum solche Kristallisatoren, die kontinuierlich betrieben werden können. Vorzugsweise sind dies die Kühlscheibenkristallisatoren oder der Kratzkühler (Diss. Poschmann, S 14). Ganz besonders bevorzugt wird zur Kristallisation ein Kratzkühler eingesetzt.

Weiterhin ist es erfindungsgemäß bevorzugt, dass das Auskristallisieren im Verfahrensschritt a) bei einer Temperatur T₁ im Bereich von Tₑ - 15°C ≤ T₁ ≤ Tₑ + 3°C (Tₑ = eutektische Temperatur der ungereinigten Phase), besonders bevorzugt bei einer Temperatur T₁ im Bereich von Tₑ - 11°C ≤ T₁ ≤ Tₑ + 2°C, noch mehr bevorzugt bei einer Temperatur T₁ im Bereich von Tₑ - 7°C ≤ T₁ ≤ Tₑ + 1°C und am meisten bevorzugt bei einer Temperatur T₁ im Bereich von Tₑ - 3°C ≤ T₁ ≤ Tₑ erfolgt.

Im Falle von Acrylsäure als Zielprodukt erfolgt gemäß der ersten besonderen Ausführungsform der im erfindungsgemäßen Verfahren enthaltenen Verfahrenstufe das Auskristallisieren im Verfahrensschritt a) bei einer Temperatur T₁ in einem Bereich von -8°C bis -26°C, noch mehr bevorzugt in einem Bereich von -9°C bis -22°C, noch mehr bevorzugt in einem Bereich von -10°C bis -18°C und am meisten bevorzugt in einem Bereich von -11 °C bis -14°C. Im Falle von Methacrylsäure als Zielprodukt erfolgt gemäß der zweiten besonderen Ausführungsform der im erfindungsgemäßen Verfahren enthaltenen Verfahrenstufe das Auskristallisieren im Verfahrensschritt a) bei einer Temperatur T₁ in einem Bereich von +6°C bis -26°C, noch mehr bevorzugt in einem Bereich von +2°C bis -16°C und am meisten bevorzugt in einem Bereich von 0°C bis -10°C.

Im Schritt b) der im erfindungsgemäßen Verfahren enthaltenen Verfahrensstufe werden die im Verfahrensschritt a) erhaltenen Kristalle, welche als eine Mischung aus Kristallen des Zielproduktes und Wasser-Kristallen vorliegen, von der Mutterlauge abgetrennt, wobei es auch hier vorteilhaft ist, zur Abtrennung der Kristalle von der Mutterlauge eine Vorrichtung zu verwenden, mittels derer dieses Abtrennen kontinuierlich durchgeführt werden kann. Für den Fall der Schichtkristallisation oder der statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer besonderen Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Erfindungsgemäß besonders bevorzugt ist der Einsatz einer Waschkolonne, in der die Abtrennung der Mutterlauge gemäß Verfahrensschritt b) und das Waschen mit den zumindest teilweise aufgeschmolzenen Kristallen gemäß Verfahrensschritt d) in einem Apparat erfolgen. Eine solche Waschkolonne weist vorteilhafterweise einen Trennbereich auf, in dem die Kristalle des Zielproduktes und die Wasser-Kristalle gewaschen werden. Für den erfolgreichen Betrieb einer Waschkolonne ist es vorteilhaft, dass die zu waschenden Kristalle hart genug sind und eine bestimmte enge Größenverteilung aufweisen, um eine ausreichende Porosität, Permeabilität und Stabilität sowie eine möglichst geringe Kompressibilität des entstehenden gepackten oder ungepackten Filterbettes zu gewährleisten.

Im Prinzip kann für das erfindungsgemäße Verfahren jedwede Waschkolonne eingesetzt werden, die die kontinuierliche Fahrweise der erfindungsgemäßen Aufreinigung oder aber auch die quasi-kontinuierliche Fahrweise, wie sie beispielsweise in den Waschkolonnen mit mechanischem Betttransport der Fa. Niro Process Technology B.V., in s'Hertogenbusch (Niederlande), erfolgt, zulässt. Bei einer üblichen Ausführungsform wird die Suspension in einer hydraulischen Waschkolonne in einem beliebigen Teilbereich der Kolonne, beispielsweise im oberen Teil der Kolonne, aufgegeben; die Mutterlauge wird über ein Filter aus der Kolonne abgezogen, wodurch sich ein dichtgepacktes Kristallbett bildet. Das sich oberhalb der Filtereinheit bereits ausgebildete Kristallbett wird von der Mutterlauge und von rezyklierter Mutterlauge in Richtung der der Zuführung gegenüberliegenden Seite der Kolonne durchströmt und durch den Strömungswiderstand in diese Richtung gedrückt. Eine solche Waschkolonne und deren Funktionsweise ist beispielsweise in der WO-A-2004/026429 beschrieben, deren Offenbarungsgehalt hinsichtlich des Aufbaus und der Funktionsweise einer Waschkolonne hiermit als Referenz eingeführt wird und einen Teil der Offenbarung der vorliegenden Anmeldung bildet.

Bei einer mechanischen Waschkolonne - beispielhaft sei auch auf die EP-A-0 193 226 B verwiesen - wird ein dichtes Kristallbett innerhalb der Kolonne mittels eines für die Schmelze durchlässigen Kolbens erzeugt. Der Kolben kann sich am oberen oder unteren Ende der Kolonne befinden; im ersten Fall erfolgt der Zulauf der Suspension im oberen Bereich der Kolonne, im zweiten Fall im mittleren oder unteren Bereich. Der Kolben ist für die Schmelze durchlässig, so dass beim Komprimieren Schmelze an der Rückseite des Kolbens austritt und dort abgezogen wird.

Ausführungsformen zur Suspensionskristallisation mit nachgeschalteter Wäsche der Kristalle in einer hydraulischen oder mechanischen Waschkolonne sind dem Buch *"*Melt Crystallization Technoloy" von G.F. Arkenbout, Technomic Publishing Co. Inc., Lancaster-Basel (1995), S. 265-288 sowie dem sich auf die Niro-Gefrierkonzentration zur Abwasservorkonzentrierung richtenden Artikel in Chemie Ingenieur Technik (72) (10/2000), 1231-1233 zu entnehmen.

Im Schritt c) der im erfindungsgemäßen Verfahren enthaltenen Verfahrensstufe wird mindestens ein Teil der abgetrennten Kristalle zu einer Schmelze aufgeschmolzen. Dieses kann dadurch geschehen, dass ein Teil der Kristalle nach der Abtrennung von der Mutterlauge aufgeschmolzen wird oder aber, was erfmdungsgemäß bevorzugt ist, dadurch, dass ein Teil der Kristalle resuspendiert und die Kristalle in der so erhaltenen Suspension aufgeschmolzen werden.

Besonders bevorzugt erfolgt das Aufschmelzen im Verfahrensschritt c) im Falle eines Einsatzes einer Waschkolonne dergestalt, dass eine sich bewegende, vorzugsweise rotierende Kratzvorrichtung oder ein rotierender Kratzer, welcher an der der Zuführung gegenüberliegenden Seite der Kolonne angebracht ist, aus Kristallen des dichtgepackten Kristallbetts und der gemäß Verfahrensschritt d) eingebrachten Waschschmelze wieder eine Suspension erzeugt. Diese Suspension wird vorzugsweise durch einen Aufschmelzer, vorzugsweise einen Wärmetauscher, gepumpt und aufgeschmolzen. Ein Teil der Schmelze kann z.B. als Waschschmelze dienen; diese wird dann in die Kolonne zurückgepumpt und wäscht vorzugsweise das in entgegengesetzter Richtung wandernde Kristallbett aus, d.h. das kristallisierte Zielprodukt und das kristallisierte Wasser werden im Gegenstrom von der zurückgeführten Schmelze aus Wasser und dem Zielprodukt gewaschen. Die Waschschmelze bewirkt einerseits ein Waschen der Kristalle, andererseits kristallisiert die Schmelze auf den Kristallen zumindest teilweise aus. Die freiwerdende Kristallisationsenthalphie erwärmt das Kristallbett im Waschbereich der Kolonne. Damit wird eine Aufreinigung zum einen durch das Waschen der Oberfläche der Wasser- und der Zielprodukt-Kristalle mit aufgeschmolzenen Wasser und aufgeschmolzenen Zielprodukt (also mit einer aufgeschmolzenen Phase beinhaltend Wasser und Zielprodukt) bewirkt, zum anderen wird durch die Kristallisation der aufgeschmolzenen Phase aus Zielprodukt und Wasser auf den bereits vorhandenen Kristallen des Zielproduktes und den bereits vorhandenen Wasser-Kristallen ein Ausheilen bzw. Ausschwitzen von Verunreinigungen erreicht. Dieses erlaubt die Herstellung einer hochreinen, wässrigen Phase des Zielproduktes (also einer wässrigen Phase, welche nahezu ausschließlich aus Wasser und Zielprodukt besteht).

Im Zusammenhang mit dem Verfahrensschritt c) ist es insbesondere bevorzugt, dass das Aufschmelzen bei einer Temperatur im Bereich von T₁ < T₂ ≤ T₂ + 20°C, besonders bevorzugt bei einer Temperatur im Bereich von T₁ <T₂ ≤T₂ + 10°C, noch mehr bevorzugt bei einer Temperatur im Bereich von T₁ < T₂ ≤ T₂ + 5°C und am meisten bevorzugt bei einer Temperatur im Bereich von T₁ <T₂≤T₂ + 1°C erfolgt. Dabei ist es insbesondere bevorzugt, dass das Aufschmelzen im Verfahrensschritt c) bei einer Temperatur T₂, die 0,1 bis 15 Grad, besonders bevorzugt 0,5 bis 10 Grad, noch mehr bevorzugt 1 bis 5 Grad und am meisten bevorzugt 2 bis 4 Grad oberhalb der Temperatur T₁ liegt, erfolgt.

Im Falle von Acrylsäure als Zielprodukt erfolgt gemäß der ersten besonderen Ausführungsform der im erfindungsgemäßen Verfahren enthaltenen Verfahrenstufe das Aufschmelzen im Verfahrensschritt c) bei einer Temperatur T₂ in einem Bereich oberhalb von -11°C bis 9°C, besonders bevorzugt in einem Bereich oberhalb von -11°C bis -1°C, noch mehr bevorzugt in einem Bereich oberhalb von -11°C bis -6°C und am meisten bevorzugt in einem Bereich oberhalb von -11°C bis -10°C. Im Falle von Methacrylsäure als Zielprodukt erfolgt gemäß der zweiten besonderen Ausführungsform der im erfindungsgemäßen Verfahren enthaltenen Verfahrenstufe das Auskristallisieren im Verfahrensschritt a) bei einer Temperatur T₁ in einem Bereich von +7°C bis -25°C, noch mehr bevorzugt in einem Bereich von +3°C bis -15°C und am meisten bevorzugt in einem Bereich von 1°C bis -9°C.

Im Schritt d) der im erfindungsgemäßen Verfahren enthaltenen Verfahrensstufe wird ein Teil der Schmelze dem Schritt b) zurückgeführt, wobei dieses Zurückführen insbesondere auf die vorstehend beschriebene Verfahrensweise erfolgt, bei der am unteren Teil der Waschkolonne aus den Kristallen des Kristallbetts und der im Verfahrensschritt c) erhaltenen Waschschmelze wieder eine Suspension erzeugt, wird, diese Suspension sodann durch einen Aufschmelzer, vorzugsweise einen Wärmetauscher, gepumpt und aufgeschmolzen wird und ein Teil der Schmelze dann als Waschschmelze dient, die in die Kolonne zurückgepumpt wird und das in entgegengesetzter Richtung wandernde Kristallbett auswäscht.

In einer speziellen Ausgestaltung des erfindungsgemäßen Verfahrens ist der Rückführstrom, der sich aus der Zurückführung der aufgeschmolzenen Kristalle aus dem Schritt c) in den Schritt b) ergibt, größer als ein Feedstrom der ungereinigten Phase, der von außen kontinuierlich dem Schritt a) zugeführt wird. Insbesondere ist der Rückführstrom mindestens genauso groß, vorzugsweise mindestens doppelt so groß, noch mehr bevorzugt mindestens viermal so groß und am meisten bevorzugt mindestens zehnmal so groß wie der Feedstrom. Durch den großen Rückführstrom wird sichergestellt, dass die thermische Belastung des Zielproduktes am Aufschmelzer verringert wird.

Zur Impfung des zu kristallisierenden Zielproduktes kann es sich als vorteilhaft erweisen, das abgetrennte, kristallisierte Zielprodukt aus Schritt b) zumindest teilweise in den Schritt a) zuführen. Die zurückgeführten Zielprodukt-Kristalle erleichtern das Kristallwachstum im Schritt a) und unterstützen so die Trennung des Zielproduktes von der Mutterlauge. Dieses ist insbesondere bei einer stark wässrigen Zusammensetzung vorteilhaft.

Grundsätzlich ist aufgrund energetischer Überlegungen ein einstufiges Aufreinigungsverfahren mit nur einer Verfahrensstufe besonders vorteilhaft und damit besonders bevorzugt. Unter Umständen ist jedoch ein zweistufiges Aufreinigungsverfahren sinnvoll. Hinsichtlich der Durchführung eines zwei- oder mehrstufigen Aufreinigungsverfahrens wird insbesondere auf die WO-A-03/078378 verwiesen, deren Offenbarungsgehalt hinsichtlich eines Aufreinigungsverfahrens beinhaltend zwei Verfahrensstufen mit den Verfahrensschritten a) bis d) hiermit als Referenz eingeführt wird und einen Teil der Offenbarung der vorliegenden Erfindung bildet.

In einer besonderen Ausgestaltung des erfindungsgemäßen Verfahrens wird die gereinigte Phase in einem weiteren Reinigungsverfahren gereinigt, insbesondere Reinigungsverfahren, bei denen gegebenenfalls das in der gereinigten Phase enthaltene Wasser zumindest teilweise vom Zielprodukt abgetrennt werden kann. Geeignete Reinigungsverfahren sind dem Fachmann hinlänglich bekannt. Vorzugsweise kommen als solche Verfahren im Einzelnen folgende zur Anwendung:
1. Einfache Destillation
   Die Reinigung einer Zusammensetzung hauptsächlich bestehend aus Wasser und Zielprodukt zum Zwecke des Abtrennens des Wassers wird in einem Großteil der Fälle mittels Azeotroprektifikation vorgenommen. Zur Anwendung kommen beispielsweise Schleppmittel wie Toluol oder MIBK (siehe EP 0 695 736 B1).
2. Extraktion des Zielproduktes
   Mit geeigneten Extraktionsmitteln kann das Zielprodukt extraktiv gewonnen werden. Die Extraktion organischer Verbindungen aus wässrigen Zusammensetzungen ist Stand der Technik. Extrahiert werden können Zielprodukte wie Acrylsäure oder Methacrylsäure beispielsweise mit flüssigen Ionenaustauschern, Mischungen aus tri-n-Alkylaminen und aliphatischen Alkoholen oder n-Butanol (Vogel et al.: Chem.Eng.Technol.23 (2000)1, Seiten 70-74; Tamada et al. in "Solvent Extraction", 1990, Herausgeber: T. Sekine, Elsevier Science Publishers B.V., Seiten 1791 - 1796; JP 57 095 938; WO-A-98/40342; Informationsbroschüre der Firma SULZER Chemtech zur fraktionierten Extraktion von (Meth)Acrylsäure mit n-Butanol).
3. Entwässerng des Zielproduktes durch Pervaporation
   Dieses Verfahren ist unter anderem in DE 44 01 405 A1 offenbart.
4. Entwässerung des Zielproduktes durch Kristallisation
   Weiterhin kann die durch das erfindungsgemäße Verfahren erhältliche aufgereinigte Phase beinhaltend Wasser und das Zielprodukt auch durch weitere Kristallisationsschritte, insbesondere auch durch Kristallisationsschritte gemäß dem erfindungsgemäßen Verfahren, von zumindest einem Teil des Wassers befreit werden.

Es sei jedoch an dieser Stelle noch einmal betont, dass insbesondere im Falle von Zielprodukten, welche später bei der Weiterverarbeitung zu Nachfolgeprodukten ebenfalls in Form wässriger Lösungen eingesetzt werden, weitere Aufreinigungsschritte, insbesondere eine Abtrennung von Wasser, üblicherweise entbehrlich sind, Durch das erfindungsgemäße Kristallisationsverfahren können Verunreinigungen bereits so stark abgereichert werden, dass sich die durch das erfindungsgemäße Verfahren erhältliche, wässrige Phase unmittelbar zur Herstellung von Nachfolgeprodukten einsetzen lässt.

Einen Beitrag zur Lösung der eingangs genannten Aufgaben leistet auch ein Verfahren zur Herstellung einer aufgereinigten Phase beinhaltend ein Zielprodukt, vorzugsweise (Meth)Acrylsäure, und Wasser, beinhaltend die Verfahrensschritte:
(α1) Bereitstellen einer ungereinigten, wässrigen Phase beinhaltend das Zielprodukt, Wasser sowie vom Zielprodukt und von Wasser verschiedene Verunreinigungen;
(α2) Aufreinigung der ungereinigten, wässrigen Phase durch das vorstehend beschriebene Verfahren zur Aufreinigung von einer ungereinigten Phase beinhaltend das Zielprodukt, Wasser und mindestens eine vom Zielprodukt und Wasser verschiedene Verunreinigung.

Im Verfahrensschritt (α1) wird zunächst eine ungereinigte, wässrige Phase beinhaltend das Zielprodukt, Wasser sowie vom Zielprodukt und von Wasser verschiedene Verunreinigungen hergestellt. Grundsätzlich kommen hierbei alle dem Fachmann bekannten Verfahren in Betracht, die, je nach Zielprodukt, letztendlich zur Bereitstellung einer solchen ungereinigten, wässrigen Phase führen. Üblicherweise handelt es sich bei dieser wässrigen Phase um irgendeine wässrige Phase, die im Verlaufe der großtechnischen Herstellung des Zielproduktes aus einer entsprechenden Ausgangsverbindung als Reaktionsgemisch anfällt. Insbesondere kommen hier wässrige Phasen in Betracht, die erhalten werden, wenn die Ausgangsverbindung in Wasser als Lösungsmittel umgesetzt wird oder aber wässrige Phasen, die erhalten werden, wenn eine Ausgangssubstanz in der Gasphase umgesetzt und das so erhaltene, gasförmige Reaktionsgemisch, welches das Zielprodukt enthält, in Wasser absorbiert wird.

Im Falle der Acrylsäure als Zielprodukt ist als Verfahrensschritt (α1) beispielsweise ein Verfahren denkbar, bei dem zunächst auf fermentativem Weg 3-Hydroxypropionsäure aus geeigneten Vorläuferverbindungen hergestellt und diese 3-Hydroxypropionsäure anschließend unter Bildung einer Roh-(Meth)Acrylsäure dehydratisiert wird. Ein solches Verfahren ist beispielsweise in WO-A-2006/022664 oder in WO-A-2005/118719 beschrieben. Denkbar ist weiterhin ein Verfahren, bei dem zunächst Glycerin unter Bildung von Acrolein dehydratisiert und das so erhaltene Acrolein zu Acrylsäure umgesetzt wird, wie dies beispielsweise in der DE-A-10 2005 028 624 beschrieben ist.

Gemäß einer bevorzugten Ausführungsform des erfindungsgemäßen Verfahrens zur Herstellung einer aufgereinigten Phase beinhaltend ein Zielprodukt und Wasser, bei dem das Zielprodukt (Meth)Acrylsäure ist, beinhaltet jedoch der Verfahrensschritt (α1) folgende Verfahrensschritte:
(β1) Herstellung einer (Meth)Acrylsäure enthaltenden Produktmischung durch Katalytische Gasphasenoxidation geeigneter C₃- bzw. C₄-Verbindungen;
(β2) Absorption der (Meth)Acrylsäure enthaltenden Produktmischung in Wasser unter erhalt einer ersten, wässrigen Phase;
(β3) gegebenenfalls mindestens teilweises Abtrennen von Wasser aus der ersten, wässrigen Phase unter Erhalt einer zweiten, wässrigen Phase.

Im Verfahrensschritt (β1) wird zunächst eine (Meth)Acrylsäure enthaltende Produktmischung durch Katalytische Gasphasenoxidation geeigneter C₃- bzw. C₄-Verbindungen hergestellt.

Erfindungsgemäß bevorzugt wird im Verfahrensschritt (β1) Acrylsäure oder Methacrylsäure durch katalytische Gasphasenoxidation von C₃- bzw. C₄-Alkanen, -Alkenen, -Alkanolen und/oder -Alkanalen und/oder Vorstufen davon hergestellt. Besonders vorteilhaft wird Acrylsäure oder Methacrylsäure durch katalytische Gasphasenoxidation von Propen, Acrolein, tert.-Butanol, Isobuten, Isobutan, Isobutyraldehyd, Methacrolein, Isobuttersäure oder Methyl-tert.-butylether hergestellt. Als Ausgangsverbindungen können alle Vorstufen der genannten Verbindungen verwendet werden, bei denen sich die eigentliche C₃-/C₄-Ausgangsverbindung erst intermediär während der Gasphasenoxidation bildet. Beispielhaft genannt für die Herstellung der Methacrylsäure sei Methyl-tert.-butylether oder Isobuttersäure.

Besonders vorteilhaft ist die katalytische Gasphasenreaktion von Propen und/oder Acrolein zu Acrylsäure mit molekularem Sauerstoff nach bekannten Verfahren, insbesondere wie sie in den Druckschriften DE-A-19 62 431, DE-A-29 43 707, DE-PS 12 05 502, EP-A-0 257 565, EP-A-0 253 409, DE-AS 22 51 364, EP-A-0 117 146, GB-PS 1 450 986 und EP-A-0 293 224 beschrieben sind. Vorzugsweise wird hierbei bei Temperaturen zwischen 200 und 450°C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch: katalytische Oxidehydrierung, wie z. B. in Catalysis Today 24 (1995), 307-313 oder US-A-5 510 558 beschrieben; durch homogene Oxidehydrierung, wie z. B. in CN-A-1 105 352 beschrieben; oder durch katalytische Dehydrierung, wie z. B. in EP-A-0 253 409, EP-A-0 293 224, DE-A-195 08 558 oder EP-A-0 117 146 beschrieben. Bei Einsatz eines Propen-/Propan-Gemischs wirkt Propan als Verdünnungsgas. Geeignete Propen-/Propan-Gemische sind auch Raffineriepropen (70% Propen und 30% Propan) oder Crackerpropen (95% Propen und 5% Propan). Grundsätzlich können Propen-/Propan-Gemische wie die o. g. mit Sauerstoff oder Luft oder einem Gemisch aus Sauerstoff und Stickstoff jeder Zusammensetzung zu Acrolein und Acrylsäure oxidiert werden.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-% inerte Verdünnungsgase. Als inerte Verdünnungsgase sind insbesondere gesättigte C1-C6-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbin-dungen mit molekularem Sauerstoff hergestellt werden. Besonders vorteilhaft ist die Methacrylsäure, z. B. durch katalytische Gasphasenoxidation von Isobuten, Isobutan, tert.-Butanol, Isobutyraldehyd, Methacrolein oder Methyl-tert.-butylether erhältlich. Als Katalysatoren werden ebenfalls übergangsmetallische Mischoxidkatalysatoren (z. B. Mo, V, W und/oder Fe) verwendet. Besonders geeignete Verfahren sind solche, bei denen die Herstellung ausgehend von Methacrolein erfolgt, insbesondere dann, wenn das Methacrolein durch gasphasenkatalytische Oxidation von tert.-Butanol, Isobutan oder Isobuten oder durch Umsetzung von Formaldehyd mit Propionaldehyd gemäß EP-B-0 092 097 oder EP-B-0 058 927 erzeugt wird. Somit besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrylsäure und (2) anschließende Oxidation des Methacroleins zu Methacrylsäure.

Ebenso wie bei der Herstellung der Acrylsäure wird nicht reine Methacrylsäure, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann. Das erfindungsgemässe Verfahren wird insbesondere dann eingesetzt, wenn das Reaktionsgemisch 0,02 bis 2 Gew.-% Methacrolein bezogen auf das gesamte Reaktionsgemisch und ansonsten im wesentlichen die gleichen entsprechenden Bestandteile wie bei der Herstellung der Acrylsäure enthält.

Im Verfahrensschritt (β2) wird die im Verfahrensschritt (β1) erhaltene, (Meth)Acrylsäure enthaltende Produktmischung in Wasser unter Erhalt einer ersten, wässrigen Phase absorbiert, wobei diese Absorption vorzugesweise in geeigneten Quenchvorrichtungen erfolgt. Vorteilhafterweise wird die Kondensation mit einem Direktkondensator durchgeführt, wobei bereits erzeugtes Kondensat mit dem heißen gasförmigen Reaktionsprodukt in Kontakt gebracht wird. Als Apparate für die Kondensation eignen sich insbesondere Sprühwäscher, Venturiwäscher, Blasensäulen oder Apparate mit berieselten Oberflächen.

Im Verfahrensschritt (β3) kann die im Verfahrensschritt (β2) erhaltene, erste wässrige Phase gegebenenfalls weiter aufgereinigt werden, wobei hier insbesondere eine Abtrennung des Wassers durch Azeotropdestillation mit geeigneten Trennmitteln, wie beispielsweise Toluol, in Betracht kommt. Nach dem Abtrennen von mindestens einem Teil des Wassers wird eine zweite wässrige Phase erhalten.

Im Verfahrensschritt (α2) wird sodann die erste wässrige Phase oder die zweite wässrige Phase als "ungereinigte Phase" im erfindungsgemäßen Verfahren zur Aufreinigung von einer ungereinigten Phase beinhaltend ein Zielprodukt, vorzugsweise (Meth)Acrylsäure, Wasser und mindestens eine vom Zielprodukt und Wasser verschiedene Verunreinigung eingesetzt.

Einen Beitrag zur Lösung der eingangs genannten Aufgabe leistet auch eine aufgereinigte Phase beinhaltend ein Zielprodukt, vorzugsweise (Meth)Acrylsäure, und Wasser, erhältlich durch das vorstehend beschriebene Verfahren.

Einen Beitrag zur Lösung der eingangs genannten Aufgaben leistet auch ein Verfahren zur Herstellung eines auf (Meth)Acrylsäure basierenden Polymers, wobei eine aufgereinigte Phase beinhaltend (Meth)Acrylsäure und Wasser, hergestellt durch das vorstehend beschriebene Verfahren zur Herstellung einer aufgereinigten Phase beinhaltend (Meth)Acrylsäure als Zielprodukt und Wasser, oder aber eine gereinigte Phase, erhältlich durch das eingangs beschriebene Verfahren zur Aufreinigung von einer ungereinigten Phase beinhaltend (Meth)Acrylsäure als Zielprodukt, Wasser und mindestens eine von (Meth)Acrylsäure und Wasser verschiedene Verunreinigung polymerisiert wird. Die Polymerisation erfolgt vorzugsweise als Lösungspolymerisation, wobei die Reaktionsführung in einem Muldenband besonders bevorzugt ist. Hierbei wird entweder die aufgereinigte Phase beinhaltend (Meth)Acrylsäure als Zielprodukt und Wasser unmittelbar eingesetzt oder gegebenenfalls zuvor entsprechend verdünnt. Im Allgemeinen erfolgt die Polymerisation in einem Medium im Falle von Acrylsäure bei einem Feststoffgehalt (mit Feststoff sind alle von Wasser und gelösten Gasen verschiedene Komponenten der Monomerlösung gemeint) in einem Bereich von 20 bis 50 Gew.-%, besonders bevorzugt in einem Bereich von 30 bis 40 Gew.-%.

Gemäß einer bevorzugten Ausführungsform dieses erfindungsgemäßen Verfahrens zur Herstellung eines auf (Meth)Acrylsäure basierenden Polymers wird die bei der Polymerisation eingesetzte, aufgereinigte Phase vor der Polymerisation nicht weiter aufgereinigt. Insbesondere wird aus dieser Phase vor der Durchführung der Polymerisation vorzugsweise weniger als 20 Gew.-%, besonders bevorzugt weniger als 10 Gew.-%, noch mehr bevorzugt weniger als 5 Gew.-% und am meisten bevorzugt überhaupt kein Wasser abgetrennt.

Weitere Einzelheiten und vorteilhafte Weiterbildungen der vorliegenden Erfindung werden anhand der folgenden Zeichnung, die die Erfindung exemplarisch veranschaulichen soll, und anhand eines Beispiels näher erläutert.

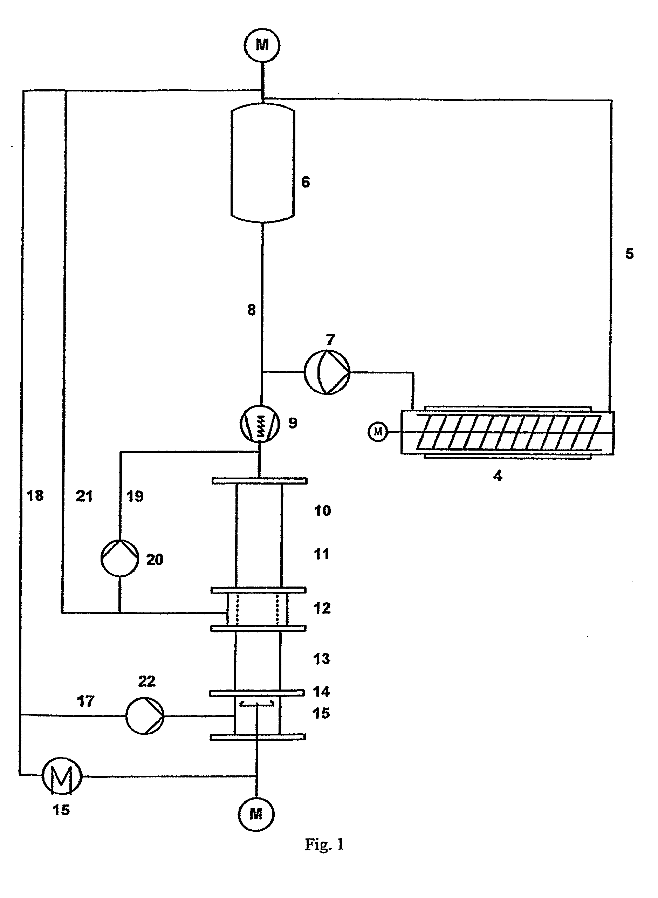

Es zeigt die Fig. 1 schematisch eine Versuchsanlage zur Durchführung des erfindungsgemäßen Aufreinigungsverfahrens.

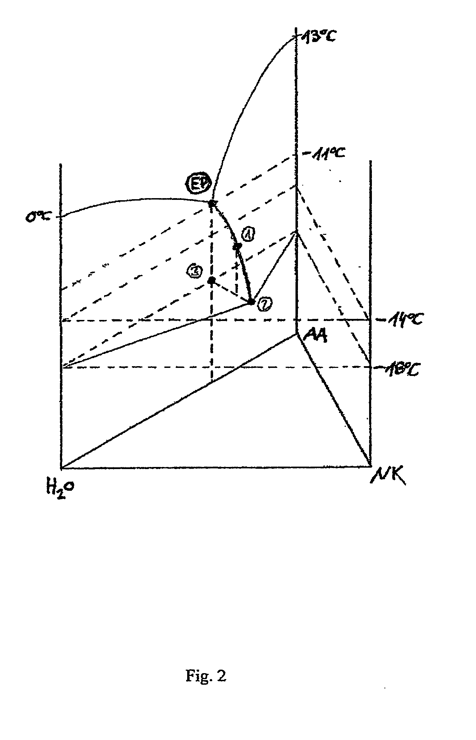

Es zeigt die Figur 2 das Phasendiagramm für die im Beispiel 1 eingesetzte, Pseudo-ternäre Zusammensetzung

### BEISPIEL:

Es wird eine Pseudo-ternäre Mischung aus 59,4 Gew.-% Acrylsäure, 39,4 Gew.-% Wasser und 1,2 Gew.-% von Acrylsäure und Wasser verschiedenen Verunreinigungen gemäß dem erfindungsgemäßen Verfahren aufgereinigt, wobei die in der Figur 1 dargestellte Versuchanordnung eingesetzt wurde.

Die eingesetzte hydraulische Waschkolonne verfügte über einen Außenmantelfilter (12) entsprechend einer dem Fachmann bekannten Philipps Waschkolonne und über eine mechanisch rotierende Kristallabnahme (14) entsprechenden einer dem Fachmann bekannten TNO-Waschkolonne. Die installierte Waschkolonne wies einen Innendurchmesser von 82 mm, einen Zuführungs- (10) und Sedimentationsbereich (11) von insgesamt 500 mm und einen Waschbereich (13) von 300 mm Länge auf. Die Feedzührung erfolgte über eine zwangsfordernde Schneckenpumpe (9). Mit Hilfe einer Filtratumwälzpumpe (20) wurde das Filtrat zurück in den Zuführungsbereich (10) der Waschkolonne gefördert. Durch den beim Durchströmen des vor der Filtrationseinheit (12) ausgebildeten Kristallbetts aufbauenden Differenzdruck wurde die notwendige Kraft zum Transport des Kristallbetts aufgebracht. Der maximale Filtratumwälzstrom (19) der Filtratumwälzpumpe (20) betrug 2,5 m³/h. Der Aufschmelzbereich (15) war mit einem Wärmetauscher (16) mit einer Austauschfläche von 1 m² und einer Umwälzpumpe (17) mit einer maximalen Förderleistung von 1 m³/h zur Rückführung der Produktschmelze (17) in den Aufschmelzbereich (15) ausgerüstet.

Eine wässrige Acrylsäuremischung beinhaltend Acrylsäure, Wasser und Nebenkomponenten entsprechend der Zusammensetzung aus Tabelle 1 wurde mit Hilfe eines Kratzkristallers (4) der Firma Armstrong auf eine Temperatur von -14°C heruntergekühlt. Bei Unterschreiten von -14°C wurden erste Kristalle beobachtet, dies entspricht Punkt 1 im pseudo-ternären Phasendiagramm, das schematisch in Figur 2 dargestellt ist. Durch eine weitere Temperaturerniedrigung auf -18°C konnte eine Suspension (5) mit einem Feststoffanteil von ca. 33 Gew.-% erzeugt werden. Zur Reifung der Kristalle wurde die Suspension mit Hilfe einer Umwälzpumpe (7) zurück in einen Verweilzeitbehälter (5) eingefahren. Entsprechend des Phasendiagramms lagen bei dieser Temperatur Mutterlauge (2) und Kristallphase (3) im Gleichgewicht nebeneinander vor. Die Zusammensetzung der jeweiligen Phasen kann ebenfalls Tabelle 1 entnommen werden.

Die erzeugte Suspension (8) wird über die zwangsfordernde Schneckenpumpe (9) aus dem Verweilzeitbehälter (6) in einen ersten Bereich (10), dem Zuführungsbereich der hydraulischen Waschkolonne, einem zylindrisch geformten Hohlraum, eingefahren. In der zweiten Zone (11) sedimentierte die binäre Kristallphase zu einem kompakten Kristallbett unter Rückführung der Mutterlauge mittels einer Steuerpumpe. Das Kristallbett bestehend aus Wasserkristallen, Acrylsäurekristallen und verunreinigter Mutterlauge wurde in den nächsten Bereich (12) gefordert, im dem die Mutterlauge über den im Außenmantel installierten Filter abgetrennt wurde. Die binäre Kristallphase wurde weiter durch den zylindrischen Hohlraum gefordert und dem Waschbereich (13) zugeführt. Hier erfolgte die Kristallwäsche der binären Kristallphase mit zurückströmendem, aufgeschmolzenem Produkt im Gegenstrom. Die gewaschene binäre Kristallphase wurde über das rotierende Messer (14) vom Kristallbett abgenommen und dem Aufschmelzbereich (15) zugeführt. Im nachgeschalteten Wärmetauscher (16) erfolgte die Totalschmelze der restlichen Kristalle bei einer Temperatur von -14 °C. Ein Teil des aufgeschmolzenen Produkts (17) wurde als Waschflüssigkeit über die Schmelzkreislaufpumpe (22) zurück in den Waschbereich (13) eingefahren. Der zur Regelung der Waschfront notwendige Temperatursprung wurde durch die Gleichgewichtstemperatur am Kristallisationspunkt im pseudo-ternären Gemisch des Zuführungsbereichs (10) und dem Schmelzpunkt der aus Wasser und Acrylsäure bestehenden, binären Kristallphase, der eutektischen Temperatur, im Aufschmelzbereich (15) hervorgerufen. (siehe Figur 2).

Bei einer Feedmenge (8) von 40 kg/h und einer Suspensionsdichte von 33 % konnte eine durchschnittliche Produktmenge (18)von 13,3 kg/h einer Wasser/Acrylsäure-Mischung abgenommen werden. Als Mutterlauge (21) wurde ein Strom von 26,7 kg/h vermessen und zurück in den Verweilzeitbehälter (6) gefahren. Zum Transport des Kristallbetts durch den zylindrischen Hohlraum war ein Steuerstrom (19) von 600 kg/h notwendig. Der Steuerstrom (19) wurde über die installierte Steuerstrompumpe (21) erzeugt. Hierbei stellte sich ein maximaler Innendruck am Zuführungsbereich (10) von 4 bar ein. Über die Sedimentationszone (11) wurde ein Druckverlust von ca. 3 bar vermessen. Über den Waschbereich (13) wurde ein Waschdruck von 300 mbar gemessen. Der Temperatursprung innerhalb des Kristallbetts im Waschbereich (10), die sog. Waschfront, wurde auf eine Höhe von 150 mm oberhalb des rotierenden Messers (14) durch Regelung der Produktmenge eingestellt.

Von Feed (1), Mutterlauge (2) und Produkt (3) wurden entsprechende Proben gezogen und mittels GC-Analyse bzw. Karl-Fischer-Titration analysiert. Die Ergebnisse sind in Tabelle 1 angegeben.

**Tahelle 1 Analysen**

| | Feed (1) | Mutterlauge (2) | Produkt (3) |
|---|---|---|---|
| Acrylsäure | 59,4% | 58,2% | 61,8% |
| Wasser | 39,4% | 40,0% | 38,1% |
| Nebenkomponenten | 1,2% | 1,8% | 0,1% |

Das so erhaltene Produkt beinhaltend Wasser und Acrylsäure kann unmittelbar zur Herstellung von Polyacrylaten eingesetzt werden.

## Patentansprüche

1. Verfahren zur Aufreinigung von einer ungereinigten Phase beinhaltend ein Zielprodukt, wobei das Zielprodukt eine organische Verbindung ist, welche in einem Zwei-Komponenten-System aus Wasser und der organischen Verbindung ein eutektisches Gemisch zu bilden vermag, Wasser und mindestens eine vom Zielprodukt und von Wasser verschiedene Verunreinigung zu einer gereinigten Phase, beinhaltend eine Verfahrensstufe, welche folgende Verfahrensschritte beinhaltet:
a) das Zielprodukt und Wasser werden aus der ungereinigten Phase unter Bildung einer Suspension, beinhaltend eine Mutterlauge und Kristalle auskristallisiert, wobei dieses Auskristallisieren bei einer Temperatur T₁ im Bereich von Tₑ-15°C ≤ T₁ ≤ Tₑ + 3°C (Tₑ = eutektische Temperatur der ungereinigten Phase) erfolgt;
b) die Kristalle werden zumindest teilweise von der Mutterlauge abgetrennt;
c) mindestens ein Teil der abgetrennten Kristalle wird zu einer Schmelze aufgeschmolzen, wobei dieses Aufschmelzen bei einer Temperatur T₂ im Bereich von T₁ < T₂ ≤ T₂ + 20°C erfolgt; und
d) mindestens ein Teil der Schmelze wird dem Schritt b) zurückgeführt, wobei der nicht zurückgeführte Teil der Schmelze als eine gereinigte Phase vorliegt.

2. Das Verfahren nach Anspruch 1, wobei das Zielprodukt (Meth)Acrylsäure ist.

3. Das Verfahren nach Anspruch 2, wobei das Auskristallisieren im Verfahrensschritt a) im Falle von Acrylsäure als Zielprodukt bei einer Temperatur T₁ in einem Bereich von -8°C bis -26°C und im Falle von Methacrylsäure als Zielprodukt bei einer Temperatur T₁ in einem Bereich von +6°C bis -26°C erfolgt.

4. Das Verfahren nach Anspruch 2, wobei das Auskristallisieren im Verfahrensschritt a) im Falle von Acrylsäure als Zielprodukt bei einer Temperatur T₁ in einem Bereich von -9°C bis -22°C und im Falle von Methacrylsäure als Zielprodukt bei einer Temperatur T₁ in einem Bereich von 2°C bis -16°C erfolgt.

5. Das Verfahren nach Anspruch 2, wobei das Auskristallisieren im Verfahrensschritt a) im Falle von Acrylsäure als Zielprodukt bei einer Temperatur T₁ in einem Bereich von -10°C bis -18°C und im Falle von Methacrylsäure als Zielprodukt bei einer Temperatur T₁ in einem Bereich von 0°C bis -10°C erfolgt.

6. Das Verfahren nach einem der vorhergehenden Ansprüche, wobei das Aufschmelzen im Verfahrensschritt c) bei einer Temperatur T₂, die 0,1 bis 15 Grad oberhalb der Temperatur T₁ liegt, erfolgt.

7. Das Verfahren nach einem der vorhergehenden Ansprüche, wobei die ungereinigte Phase einen Gehalt an vom Zielprodukt und Wasser verschiedenen Verunreinigungen von höchstens 10 Gew.-%, bezogen auf die ungereinigte Phase, aufweist.

8. Verfahren nach einem der vorhergehenden Ansprüche, wobei die Kristalle im Gegenstrom von der zurückgeführten Schmelze gewaschen werden.

9. Verfahren nach einem der vorhergehenden Ansprüche, wobei die gereinigte Phase in einem weiteren Reinigungsverfahren gereinigt wird.

10. Verfahren nach einem der vorhergehenden Ansprüche, wobei die nach dem Schritt b) abgetrennte Mutterlauge zumindest teilweise in den Schritt a) zurückgeführt wird.

11. Verfahren nach einem der vorhergehenden Ansprüche zur Herstellung einer aufgereinigten Phase beinhaltend ein Zielprodukt und Wasser, beinhaltend die Verfahrensschritte
(α1) Bereitstellen einer ungereinigten, wässrigen Phase beinhaltend das Zielprodukt, Wasser sowie vom Zielprodukt und von Wasser verschiedene Verunreinigungen;
(α2) Aufreinigung der ungereinigten, wässrigen Phase durch ein Verfahren nach einem der Ansprüche 1 bis 10 unter Erhalt einer aufgereinigten Phase beinhaltend das Zielprodukt und Wasser.

12. Das Verfahren nach Anspruch 11, wobei das Zielprodukt (Meth)Acrylsäure ist.

13. Verfahren nach Anspruch 12, wobei Verfahrensschritt (α1) folgenden Verfahrensschritte beinhaltet:
(β1) Herstellung einer (Meth)Acrylsäure enthaltenden Produktmischung durch katalytische Gasphasenoxidation geeigneter C₃- bzw. C₄-Verbindungen;
(β2) Absorption der (Meth)Acrylsäure enthaltenden Produktmischung in Wasser unter Erhalt einer ersten, wässrigen Phase;
(β3) gegebenenfalls mindestens teilweises Abtrennen von Wasser aus der ersten, wässrigen Phase unter Erhalt einer zweiten, wässrigen Phase.

14. Ein Verfahren zur Herstellung eines auf (Meth)Acrylsäure basierenden Polymers, wobei eine mit einem Verfahren gemäß einem der Ansprüche 11 bis 13 aufgereinigte Phase beinhaltend (Meth)Acrylsäure und Wasser oder eine mit einem Verfahren gemäß einem der Ansprüche 1 bis 10 gereinigte Phase beinhaltend (Meth)Acrylsäure und Wasser, polymerisiert wird.

15. Das Verfahren nach Anspruch 14, wobei die aufgereinigte Phase vor der Polymerisation nicht weiter aufgereinigt wird.

## Claims

1. Process for the purification of an impure phase comprising a target product, the target product being an organic compound which, in a two-component system comprising water and the organic compound, is capable of forming a eutectic mixture, water and at least one impurity differing from the target product and from water to give a purified phase, comprising a process stage which comprises the following process steps:
a) the target product and water are crystallized out of the impure phase with formation of a suspension comprising a mother liquor and crystals, this crystallization being effected at a temperature T₁ in the range of Tₑ - 15°C ≤ T₁ Tₑ + 3°C (Tₑ = eutectic temperature of the impure phase);
b) the crystals are at least partly separated from the mother liquor;
c) at least a part of the crystals separated off is melted to give a melt, this melting being effected at a temperature T₂ in the range of T₁ < T₂ ≤ T₂ + 20°C; and
d) at least a part of the melt is recycled to step b), the non-recycled part of the melt being present as a purified phase.

2. Process according to Claim 1, the target product being (meth)acrylic acid.

3. Process according to Claim 2, the crystallization in process step a) being effected at a temperature T₁ in a range from -8°C to -26°C in the case of acrylic acid as target product and at a temperature T₁ in a range from +6°C to -26°C in the case of methacrylic acid as target product.

4. Process according to Claim 2, the crystallization in process step a) being effected at a temperature T₁ in a range from -9°C to -22°C in the case of acrylic acid as target product and at a temperature T₁ in a range from 2°C to -16°C in the case of methacrylic acid as target product.

5. Process according to Claim 2, the crystallization in process step a) being effected at a temperature T₁ in a range from -10°C to -18°C in the case of acrylic acid as target product and at a temperature T₁ in a range from 0°C to -10°C in the case of methacrylic acid as target product.

6. Process according to any of the preceding claims, the melting in process step c) being effected at a temperature T₂ which is 0.1 to 15 degrees above the temperature T₁.

7. Process according to any of the preceding claims, the impure phase having a content of not more than 10% by weight, based on the impure phase, of compounds differing from the target product and water.

8. Process according to any of the preceding claims, the crystals being washed countercurrently by the recycled melt.

9. Process according to any of the preceding claims, the purified phase being purified in a further purification process.

10. Process according to any of the preceding claims, the mother liquor separated off after step b) being at least partly recycled to step a).

11. Process according to any of the preceding claims for the preparation of a purified phase comprising a target product and water, comprising the process steps
(α1) provision of an impure, aqueous phase comprising the target product, water and impurities differing from the target product and from water;
(α2) purification of the impure, aqueous phase by a process according to any of Claims 1 to 10 to give a purified phase comprising the target product and water.

12. Process according to Claim 11, the target product being (meth)acrylic acid.

13. Process according to Claim 12, process step (α1) comprising the following process steps:
(β1) preparation of a product mixture containing (meth)acrylic acid by catalytic gas-phase oxidation of suitable C₃ or C₄ compounds;
(β2) absorption of the product mixture containing (meth)acrylic acid in water to give a first, aqueous phase;
(β3) optionally at least partial separation of water from the first, aqueous phase to give a second, aqueous phase.

14. Process for the preparation of a polymer based on (meth)acrylic acid, a phase purified by a process according to any of Claims 11 to 13 and comprising (meth)acrylic acid and water or a phase purified by a process according to any of Claims 1 to 10 and comprising (meth)acrylic acid and water being polymerized.

15. Process according to Claim 14, the purified phase not being further purified prior to the polymerization.

## Revendications

1. Procédé pour la purification d'une phase non purifiée contenant un produit cible, le produit cible étant un composé organique qui peut former un mélange eutectique dans un système à deux composants d'eau et du composé organique, de l'eau et au moins une impureté différente du produit cible et de l'eau en une phase purifiée, comprenant un étage de procédé qui comprend les étapes de procédé suivantes :
a) le produit cible et l'eau sont séparés par cristallisation de la phase non purifiée avec formation d'une suspension, comprenant une lessive mère et des cristaux, cette séparation par cristallisation ayant lieu à une température T₁ dans la plage de Tₑ - 15C ≤ T₁ ≤ Tₑ + 3°C (Tₑ = température eutectique de la phase non purifiée) ;
b) les cristaux sont séparés au moins partiellement de la lessive mère ;
c) au moins une partie des cristaux séparés est fondue en une masse fondue, cette fusion ayant lieu à une température T₂ dans la plage de T₁ < T₂ ≤ T₂ + 20°C ; et
d) au moins une partie de la masse fondue est recyclée dans l'étape b), la partie non recyclée de la masse fondue se trouvant sous forme de phase purifiée.

2. Procédé selon la revendication 1, le produit cible étant l'acide (méth)acrylique.

3. Procédé selon la revendication 2, la séparation par cristallisation dans l'étape de procédé a), dans le cas de l'acide acrylique comme produit cible, ayant lieu à une température T₁ dans une plage de -8°C à -26°C et, dans le cas de l'acide méthacrylique comme produit cible, à une température T₁ dans une plage de +6°C à -26°C.

4. Procédé selon la revendication 2, la séparation par cristallisation dans l'étape de procédé a), dans le cas de l'acide acrylique comme produit cible, ayant lieu à une température T₁ dans une plage de -9°C à -22°C et, dans le cas de l'acide méthacrylique comme produit cible, à une température T₁ dans une plage de +2°C à -16°C.

5. Procédé selon la revendication 2, la séparation par cristallisation dans l'étape de procédé a), dans le cas de l'acide acrylique comme produit cible, ayant lieu à une température T₁ dans une plage de -10°C à -18°C et, dans le cas de l'acide méthacrylique comme produit cible, à une température T₁ dans une plage de 0°C à -10°C.

6. Procédé selon l'une quelconque des revendications précédentes, la fusion, dans l'étape de procédé c), ayant lieu à une température T₂, qui se situe 0,1 à 15 degrés au-dessus de la température T₁.

7. Procédé selon l'une quelconque des revendications précédentes, la phase non purifiée présentant une teneur en produit cible et en impuretés différentes de l'eau d'au plus 10% en poids, par rapport à la phase non purifiée.

8. Procédé selon l'une quelconque des revendications précédentes, les cristaux étant lavés à contre-courant de la masse fondue recyclée.

9. Procédé selon l'une quelconque des revendications précédentes, la phase purifiée étant purifiée dans un autre procédé de purification.

10. Procédé selon l'une quelconque des revendications précédentes, la lessive mère séparée après l'étape b) étant recyclée au moins partiellement dans l' étape a) .

11. Procédé selon l'une quelconque des revendications précédentes pour la préparation d'une phase purifiée comprenant un produit cible et de l'eau, comprenant les étapes de procédé
(α1) mise à disposition d'une phase aqueuse, non purifiée, comprenant le produit cible, de l'eau ainsi que des impuretés différentes du produit cible et de l'eau ;
(α2) purification de la phase aqueuse, non purifiée par un procédé selon l'une quelconque des revendications 1 à 10 avec obtention d'une phase purifiée comprenant le produit cible et de l'eau.

12. Procédé selon la revendication 11, le produit cible étant l'acide (méth)acrylique.

13. Procédé selon la revendication 12, l'étape de procédé (α1) comprenant les étapes de procédé suivantes :
(β1) préparation d'un mélange de produits contenant de l'acide (méth)acrylique par oxydation catalytique en phase gazeuse de composés appropriés en C₃ ou en C₄ ;
(β2) absorption du mélange de produits contenant de l'acide (méth)acrylique dans l'eau avec obtention d'une première phase aqueuse ;
(β3) le cas échéant séparation au moins partielle de l'eau de la première phase aqueuse avec obtention d'une deuxième phase aqueuse.

14. Procédé pour la préparation d'un polymère à base d'acide (méth)acrylique, une phase, purifiée par un procédé selon l'une quelconque des revendications 11 à 13, comprenant de l'acide (méth)acrylique et de l'eau ou une phase, purifiée par un procédé selon l'une quelconque des revendications 1 à 10, comprenant de l'acide (méth)acrylique et de l'eau, étant polymérisée.

15. Procédé selon la revendication 14, la phase purifiée n'étant pas purifiée davantage avant la polymérisation.
